# EUROPÄISCHE PATENTSCHRIFT

(11) **EP 1 413 253 B1**
(45) Veröffentlichungstag und Bekanntmachung des Hinweises auf die Patenterteilung: **27.04.2005**
(21) Anmeldenummer: 03011430.0
(22) Anmeldetag: 20.05.2003
(51) Int. Cl.: A61B 10/00

(54) **Biopsathaltevorrichtung für eine Biopsiekanüle**
Sample holding device for a biospy cannula
Dispositif de retenue d'un échantilon pour une canule de biopsie

(30) Priorität: 25.10.2002 DE 10250071
(43) Veröffentlichungstag der Anmeldung: 28.04.2004
(73) Patentinhaber: SOMATEX Medical Technologies GmbH, 14513 Teltow (DE)
(72) Erfinder: Dunker, Thomas, 14513 Teltow (DE); Kniep, Frank, 14979 Grossbeeren / OT Kleinbeeren (DE); Hornscheidt, Dirk, 10245 Berlin (DE); Rishmawi, Suhail, 10965 Berlin (DE)
(74) Vertreter: Haschick, Gerald, Dipl.-Ing.

(56) Entgegenhaltungen:
- US-A- 4 461 305
- US-A- 5 333 619
- US-B1- 6 443 910

## Beschreibung

Die Erfindung betrifft eine Biopsathaltevorrichtung für eine Biopsiekanüle zur Ausführung transkutaner Biopsien von Gewebe, insbesondere von hartem Gewebe und Knochenmarksgewebe, mit einer in das proximale Ende der Biopsiekanüle einführbaren Biopsathaltevorrichtung, welche zwischen der Innenwand der Biopsiekanüle und dem entnehmenden Gewebezylinder einschiebbar ist.

Der Stand der Technik stellt sich wie folgt dar. Herkömmliche Nadeln zur bioptischen, transkutanen Entnahme von hartem Gewebe, insbesondere Knochenmarksgewebe, bestehen aus einem Zylinder verschiedenster Länge und Durchmesser, dessen proximales Ende einen Griff aufweist und dessen distales Ende verjüngt ist und in einer Bohrung ausläuft, die einen Schneidrand hat. Beim Eindrücken und gleichzeitigen Drehen der Nadel um ihre Achse schneidet dieses Ende aus dem zu entnehmenden Gewebe ein zylindrisches Stück heraus, das die Nadel in ihrem hohlen Innenraum aufnimmt. Um aus den Geweben den Zylinder als Biopsie entnehmen zu können, muss anschließend die Verbindung des distalen Endes des Zylinders mit dem Rest des Gewebes, in das die Nadel eingedrungen wird, unterbrochen werden. Bei den herkömmlichen Verfahren wird zu diesem Zweck der Griff der Nadel in Schwingungen versetzt, die in einer Ebene rechtwinklig zur Nadelachse verlaufen, wobei am Eintrittspunkt in das Gewebe ein Trokar vorgesehen ist. Die Folge dieser Behandlung ist ein Bruch der Verbindungen zwischen dem distalen Ende der Biopsie und dem übrigen Gewebe in der Höhe des distalen Endes der Nadel, die herausgezogen werden kann und in ihrem Inneren das bioptische Material enthält, welches durch die Verjüngung am Ende der Nadel festgehalten wird. Diese Untersuchungsmethode hat die nachstehenden Nachteile. Häufig bleibt das bioptische Material nicht in der Nadel zurück, da es nicht vollständig vom Rest des Gewebes gelöst wird und da ferner ein wenn auch nur geringer Unterdruck innerhalb der Nadel entsteht, während diese aus dem Gewebe herausgezogen wird und das bioptische Material anschließend angesaugt wird, wobei diese Ansaugung nicht die entgegengesetzte Bremswirkung überwinden kann, die durch die Verjüngung des Nadelendes verursacht wird. All das macht die Wiederholung der gesamten bioptischen Untersuchung notwendig. Häufig führen die hier erläuterten Schwierigkeiten während des Herausziehens der Nadel dazu, dass das bioptische Material aus dem distalen Ende der Nadel herausrutscht, was eine Verletzung und einen Bruch des bioptischen Zylinders während des Rückzugsweges durch das Gewebe und den Verlust eines Teils des distalen Bereiches der Biopsie zur Folge hat. Die Bewegungen oder Schwingungen, die auf die Nadel ausgeübt werden, um die Biopsie zu lösen, bewirken häufig kleinere Frakturen an der Oberfläche und Unterbrechungen im Zusammenhalt des harten Gewebes, in das die Nadel eingeführt worden ist, wobei die Möglichkeit mit der Entfernung von der Oberfläche des Gewebes allmählich anwächst. Das hat Schmerzen für den Patienten und Beschädigungen der Nadel zur Folge, die dazu neigt, sich zu verbiegen, so dass sie ihre notwendige Geradlinigkeit verliert.

Des Weiteren ist ein Patent DE 43 05 226 "Vorrichtung für Nadeln für transkutane Biopsien" bekannt, wobei eine Zusatzvorrichtung für die herkömmlichen Biopsienadeln für die transkutane Biopsie von Gewebe, insbesondere hartem Gewebe und Knochenmarksgewebe, gegeben ist. Die Zusatzvorrichtung gestattet die störungsfreie Ausführung der Biopsie an jedem Gewebe, angefangen bei harten, kompakten Knochen bis hin zu sehr dünnem und sprödem Knochenmarksgewebe, ohne dass dabei die Gefahr besteht, dass die Biopsienadel ohne Gewebeprobe herausgezogen wird. Die Zusatzvorrichtung verringert die Gefahr von Verletzungen des Gewebes während der Biopsie, was Schmerzen des Patienten verringert. Das Grundprinzip der Zusatzvorrichtung besteht darin, dass eine einführbare Struktur am proximalen Ende der Biopsienadel eingeführt wird und sich zwischen die Innenwand der Nadel und den Gewebezylinder einschiebt. Auf Grundlage des zylindrischen Aufbaus der Biopsiekanüle wird diese Struktur zusammengeklemmt, womit das Biopsat festgehalten wird. Der Nachteil dieser technischen Lösung ist dadurch begründet, dass durch das Zusammenklemmen der Struktur das innenliegende Biopsat einer Presspassung unterliegt. Diese Presspassung verursacht Quetschartefakte des Biopsates. Schlussfolgernd daraus wird bei der nachfolgenden Untersuchung des Biopsates auf Grundlage der verursachten Quetschartefakte ein Untersuchungsergebnis möglicherweise verfälscht.

US 5,333,619 offenbart eine Biopsathaltevorrichtung gemäss dem Oberbegriff des Anspruchs 1.

Der Erfindung liegt die Aufgabe zu Grunde, eine Biopsathaltevorrichtung für die Biopsiekanüle zur Ausführung transkutaner Biopsien von Gewebe, insbesondere von hartem Gewebe und Knochenmarksgewebe, zu finden, wobei der Nachteil des Standes der Technik überwunden wird und eine Entnahme des Biopsates über die Biopsiekanüle mit äußerst geringen Quetschartefakten durchführbar wird und eine Gewähr gegeben ist, dass eine Entnahme eines Biopsates über die Biopsiekanüle garantiert ist.

Erfindungsgemäß wird die Aufgabe dadurch gelöst, dass eine Biopsathaltevorrichtung für eine Biopsiekanüle zur Ausführung transkutaner Biopsien von Gewebe, insbesondere von hartem Gewebe und Knochenmarksgewebe, realisiert ist, wobei der Patentanspruch 1 mit seinen Unteransprüchen als technische Lösung ausgeführt wird.

Dabei wird eine Biopsathaltevorrichtung in Form eines Griffstückes und ein an dem Griffstück angeordneter Draht ausgeführt. Das Griffstück ist in jedem Fall an das Griffstück der Biopsiekanüle arretierbar. Der Draht ist an seinem distalen Ende mit einem Anschluss ausgeführt, wobei gewährleistet ist, dass der Draht am distalen Ende der Biopsiekanüle an der Innenwand der Biopsiekanüle und eines Biopsatzylinders vorhanden ist. Der Draht ist mit einem Vorspannungswinkel am Griffstück mittig angeordnet, wobei der Vorspannungswinkel zwischen 1 und 90° gegeben ist. An dem Draht und der vorgegebenen Spitze mit Anschliff ist ein Anschliffwinkel von 5 bis 85°, vorteilhafterweise 20°, sinnvoll. Dabei ist der Draht mit dem Anschliff so ausgeführt, dass der Anschliff hin zum entnehmenden Biopsatzylinder gerichtet ist. Der Anschliff kann auch mit einem Hohlschliff oder einem bauchigen Schliff ausgestaltet sein. Wesentlich ist dabei, dass der Draht eine Länge aufweist, welche ausreichend ist, um eine entsprechendes Biopsat aus der Biopsiekanüle zu entnehmen. Dabei endet der Draht unmittelbar am Ende der Biopsiekanüle.

Des Weiteren ist eine Lösung gegeben, wobei ein Schaft am Griffstück angeordnet ist. Dabei wird der Schaft über das Griffstück in die Biopsiekanüle am proximalen Ende der Biopsiekanüle eingeführt. An diesem Schaft ist am distalen Ende ein Draht mit einem definierten Vorspannungswinkel angeordnet. Der Schaft für die proximale Einführung in die Biopsiekanüle weist ein Griffstück auf, welches nach Einführung in die Biopsiekanüle arretierbar mit dem Griffstück der Biopsiekanüle ausgeführt ist. Der Draht am distalen Ende des Schaftes ist in einer festen Verbindung mit einem entsprechende Vorspannungswinkel zwischen 1° bis 90° je nach Anwendungsfall tolerierend angeordnet. Der Draht weist an seinem distalen Ende eine Spitze mit einem Anschliff auf, wobei der Anschliff je nach Anwendungsfall ebenfalls von 5° bis 85°, vorteilhafterweise 20°, vorhanden ist und der Anschliff so gegeben ist, dass er hin zum Biopsiezylinder des zu entnehmenden Biopsates gerichtet ist. Die Länge des Schaftes und des dazugehörenden Drahtes ist so definiert, dass vorteilhafterweise der Draht in seiner Länge ca. 25 mm beträgt. Dabei muss die Relation der Größenordnung so eingehalten werden, dass der Schaft bei der Einführung in die Biopsiekanüle bekannter Bauart eine Länge aufweist, dass er unmittelbar am Ende der Biopsiekanüle zwischen der Innenwand der Biopsiekanüle und dem Biopsat endet. Es muss also gewährleistet sein, dass die Biopsiekanüle mit ihrem distalen Ende eine Einheit mit dem distalen Ende des Drahtes des Schaftes ergibt. Der Draht, welcher an dem Schaft fest angeordnet ist, ist vorteilhafterweise mit einer aufgerauhten Fläche gegeben. Das Profil des Drahtes ist variabel gestaltbar. Der Querschnitt des Drahtes ist so ausgeführt, dass bei der Fixierung des Schaftes in der Biopsiekanüle nur minimale Quetschartefakte zwischen der Innenwand der Biopsiekanüle und dem innenliegenden Biopsatzylinder entstehen. Vorteilhafterweise ist der Durchmesser des Drahtes ca. 0,35 mm.

Der diesseitig erläuterte Schaft mit dem am distalen Ende vorhandenen Draht zur Einführung am proximalen Ende in eine an sich bekannte Biopsiekanüle gestattet die störungsfreie Ausführung der Biopsie an jedem Gewebe, angefangen bei harten, kompakten Knochen bis hin zu sehr dünnem und sprödem Knochenmarksgewebe, ohne dass die Gefahr besteht, dass die Biopsiekanüle ohne Gewebeprobe herausgezogen wird. Durch den Schaft mit dem am distalen Ende vorhandenen Draht verringert sich die Gefahr von Verletzungen des Gewebes während der Biopsie, was Schmerzen des Patienten verringert. Schließlich wird auch die Biopsiekanüle mechanisch weniger beansprucht, da Biegungen vermieden werden, wodurch die Lebensdauer der Biopsiekanüle verlängert wird. Des Weiteren wird gewährleistet, dass das Biopsat in hoher Qualität entnommen wird, da sehr geringfügige verursachte Quetschartefakte zwischen der Innenwand der Biopsiekanüle und dem Biopsatzylinder entstehen. Somit ist eine sehr hochwertige Analyse des Biopsates auf Grundlage geringer Quetschartefakte möglich.

Weitere Vorteile und Merkmale der Erfindung ergeben sich aus den Ansprüchen und aus dem nachfolgenden Ausführungsbeispiel, in dem ein erfindungsgemäßer Schaft für eine Biopsiekanüle unter Bezugnahme auf die Zeichnung im Einzelnen erläutert ist. Das Ausführungsbeispiel zeigt dabei folgende Figuren:
- Figur 1a:: Schaft mit Griffstück
- Figur 1b:: Draht mit Griffstück
- Figur 2:: Biopsienadel
- Figur 3:: Trokar
- Figur 4:: Platzierung Biopsienadel im Biopsat
- Figur 5:: Platzierung des Schaftes in der Biopsienadel
- Figur 6 und 7:: Ansicht der Biopsienadel und der Nadel 3 mit entsprechender Drehbewegung

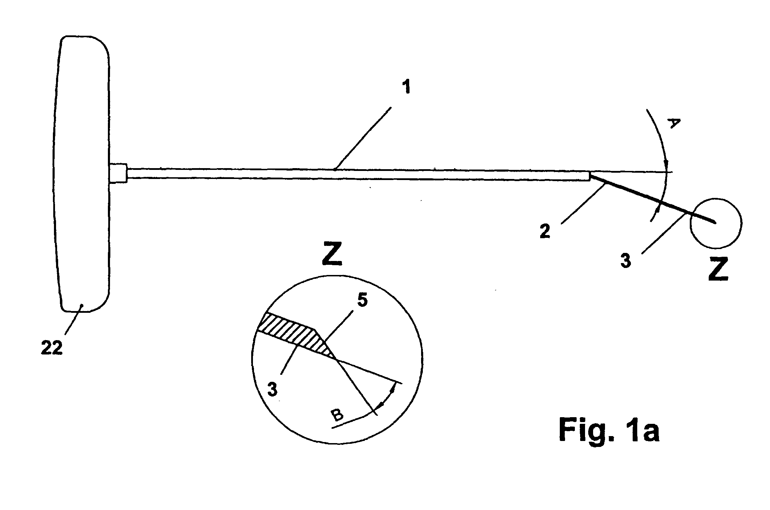

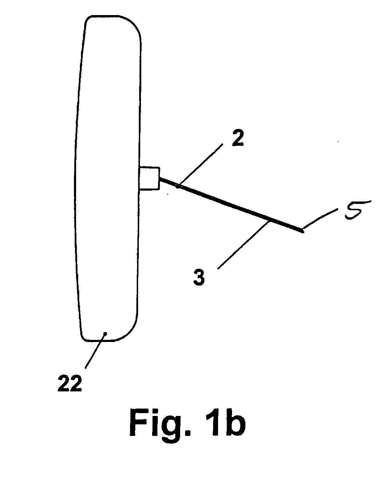

Figur 1b zeigt eine erfindungsgemäße Vorrichtung in der Art, dass ein in dem Griffstück 20 arretierbares Griffstück 22 vorhanden ist. An dem Griffstück 22 ist mittig ein Draht 3 mit einem Vorspannungswinkel 2 gegeben. Die Länge des Drahtes 3 ist so ausgeführt, dass im Wesentlichen das unmittelbare Ende der Biopsiekanüle 4 erreicht wird. Über den Vorspannungswinkel 2 ist gegeben, dass ein Gleiten des Drahtes 3 an der Innenwand der Biopsiekanüle 4 zwischen dem Biopsatzylinder 6 ausgeführt wird. Der Anschliff 5 des Drahtes 3 ist in der Art ausgeführt, dass der Anschliff 5 hin zum Biopsatzylinder 6 gegeben ist und somit ein optimales Verdrängen des Biopsatzylinders 6 bei Einführung des Drahtes 3 zwischen der Innenwand der Biopsiekanüle 4 und des Biopsatzylinders 6 möglich ist. Grundsätzlich kann man davon ausgehen, dass die Länge des Drahtes sich immer nach der Länge der entsprechenden Biopsiekanüle 4 richtet. Sollte die Biopsiekanüle 4 eine Größenordnung aufweisen, was eine korrekte Einführung des Drahtes 3 über das Griffstück 22 nicht mehr ermöglicht, wird nachfolgend die Figur 1a mit einem zusätzlich angebrachten Schaft 1 ausgeführt.

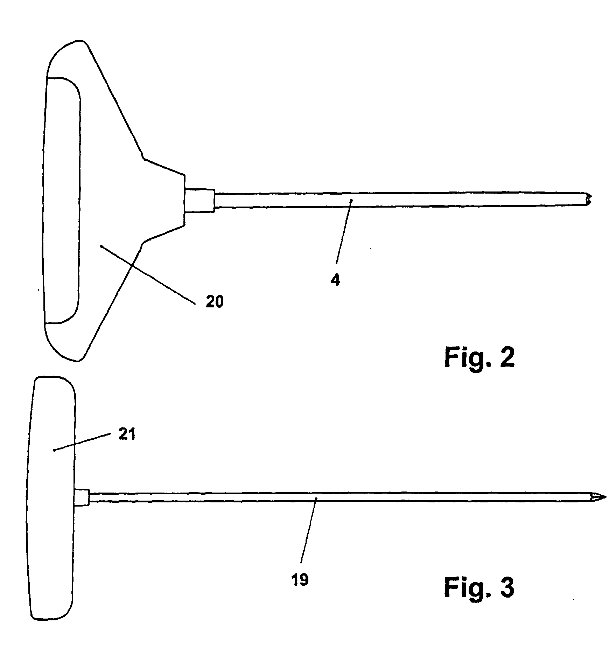

Figur 1a zeigt einen Schaft 1 mit am proximalen Ende angeordneten Griffstück 22 und am distalen Ende vorhandenen Draht 3 mit einem entsprechenden Vorspannungswinkel 2. Der Schaft 1 ist so angeordnet, dass er passgerecht in die Biopsiekanüle 4 gemäß Figur 2 einschiebbar ist. Wichtig ist dabei zu erwähnen, dass der Griff 22 des Schaftes 1 fest arretierbar in den Griff 20 der Biopsiekanüle 4 nach Einführung des Schaftes 1 ausgeführt ist. Am distalen Ende des Schaftes 1 ist ein Draht 3 mit einer Länge von 25 mm gegeben. An der Spitze des Drahtes 3 ist eine Spitze mit Anschliff 5 vorhanden, wobei der Winkel des Anschliffes B so ausgeführt ist, dass er zwischen 5° bis 85° gegeben ist, vorzugsweise in dem Ausführungsbeispiel beträgt der Anschliff B ca. 20°. Dabei ist der Anschliff 5 so gegeben, dass er bei Einführung des Schaftes 1 in die Biopsiekanüle 4 zwischen dem Biopsatzylinder 6 und der Innenwand der Biopsiekanüle 4 hin zum Biopsatzylinder 6 ausgerichtet ist. Der Vorspannungswinkel 2, welcher die Anordnung des Drahtes 3 definiert, bewegt sich in der Größenordnung von 1° bis 90°. In dem bevorzugten Ausführungsbeispiel beträgt der Vorspannungswinkel 2 ca. 10°. Dieser Vorspannungswinkel 2 wird durch entsprechende Anordnung des Drahtes 3 am distalen Ende des Schaftes 1 mit einer speziellen festen Anbauweise hervorgerufen. Durch die Anordnung des Vorspannungswinkels 2 des Drahtes 3 ist in jedem Fall eine Spannung des Drahtes 3 bei Einführung des Schaftes 1 in eine Biopsiekanüle 4 gemäß Figur 2 vorgegeben. Auf Grundlage dieser Vorspannung wird der Draht 3 des Schaftes 1 bei Einführung in die Biopsiekanüle 4 an der äußersten Kante zwischen der Innenwand der Biopsiekanüle 4 und dem beinhalteten Biopsatzylinders 6 eingeführt. Somit wird gewährleistet, dass äußerst geringe Biopsatartefakte bei der Entnahme des Biopsates über die Biopsiekanüle 4 hervorgerufen werden.

Die Figur 2 und die Figur 3 zeigen eine an sich bekannte Biopsiekanüle 4 bzw. einen zum Setzen der Biopsiekanüle 4 vorhandenen Trokar 19. Die Griffstücke 21 des Trokars 19 bzw. 22 des Schaftes 1 sind jeweils in dem Griffstück 20 der Biopsiekanüle 4 arretierbar fest positionierbar. Die Biopsiekanüle 4 der Figur 2 wird über den Trokar 19 der Figur 3 in das zu entnehmende Gewebe gesetzt.

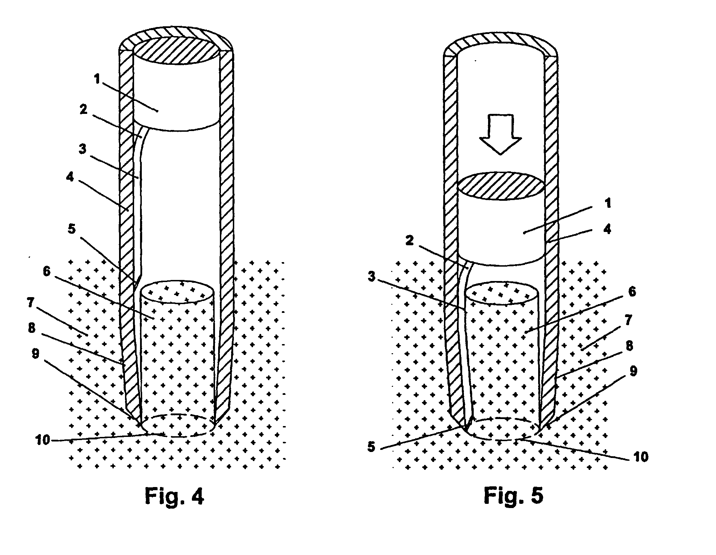

Auf Grundlage dieser Platzierung der Biopsiekanüle 4 ist im distalen Ende der Biopsiekanüle 4 ein zylindrisches Biopsat 6 im Innenraum der Biopsiekanüle 4 platziert. Diese Platzierung der Biopsiekanüle 4 wird in der Figur 4 dargestellt. Nach Platzierung der Biopsiekanüle 4 in das zu entnehmende Gewebe und dem damit vorhandenen innenliegenden Biopsiezylinder 6 wird nachfolgend der Schaft 1 über den Griff 22 mit dem am distalen Ende vorhandenen Draht 3 in die Biopsiekanüle 4 proximal eingeführt.

Durch das Setzen des Schaftes 1 bis hin zur Arretierung des Griffes 22 des Schaftes 1 in den Griff 20 der Biopsiekanüle 4 wird die Platzierung des Schaftes 1 mit dem am distalen Ende vorhandenen Draht 3 in der Figur 5 zeichnerisch dargestellt. Durch den gegebenen Vorspannungswinkel 2 und der damit vorhandenen Spannung des Drahtes 3 wird der Draht 3 durch Einführung des Schaftes 1 in die Biopsiekanüle 4 unmittelbar an der Innenwand der Biopsiekanüle 4 zwischen der Innenwand und dem Biopsatzylinder 6 getrieben. Auf Grundlage der zylindrischen Ausführung der Biopsiekanüle 4 am distalen Ende wird dadurch auch eine entsprechende Führungsspannung des Drahtes 3 vorgegeben. Die Länge des Schaftes 1 mit dem am distalen Ende gegebenen Draht 3 ist so ausgeführt, dass nach Einführung und Arretierung des Griffes 22 des Schaftes 1 in den Griff 20 der Biopsiekanüle 4 die Spitze 5 des Drahtes 3 unmittelbar am distalen Ende der Biopsiekanüle 4 endet.

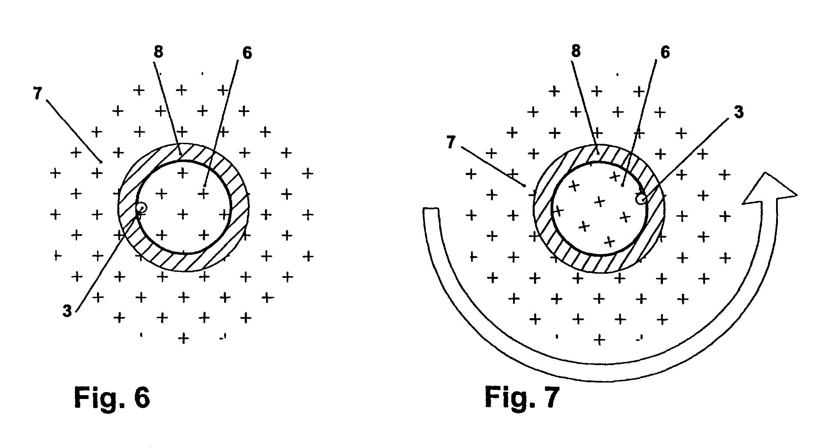

In der Figur 6 bzw. Figur 7 wird nun dargestellt, dass die Handhabung der Biopsiekanüle 4 im Folgenden so ausgeführt ist, dass durch eine Drehung der Biopsiekanüle 4 ein Abscheren des Biopsates hervorgerufen wird. Nach Drehung der Biopsiekanüle 4 mit beinhaltetem Schaft 1 wird nachfolgend die entsprechende Biopsiekanüle aus dem zu entnehmenden Gewebe herausgezogen, und über einen zusätzlich gegebenen Auswerfer wird das entsprechende Biopsat nach Entnahme des Schaftes 1 entnommen. Auf Grundlage der sehr geringen Quetschartefaktur des Biopsates zwischen der Innenwand der Biopsiekanüle 4 und des Drahtes 3 des Schaftes 1 sowie des beinhalteten Biopsiezylinders 6 ist eine hohe Qualität zur weiteren Untersuchung des Biopsates gegeben. Der wesentliche Vorteil des erfindungsgemäßen Schaftes 1 mit dem am distalen Ende vorhandenen Draht 3 ist dadurch gegeben, dass durch das Eindringen des Drahtes 3 des Schaftes 1 an der Innenwand zwischen der Innenwand und dem Biopsatzylinder 6 ein Druck erzeugt wird, welcher gewährleistet, dass der Biopsatzylinder 6 nach Drehung der Biopsiekanüle 4 in jedem Fall am distalen Ende der Biopsiekanüle 4 festgehalten wird und somit eine hohe Gewährleistung einer Beinhaltung eines Biopsates bei Entnahme der Biopsiekanüle 4 gegeben ist.

### Bezugszeichen

- 1: Schaft
- 2: Vorspannungswinkel
- 3: Draht
- 4: Biopsiekanüle
- 5: Spitze mit Anschliff
- 6: Biopsatzylinder
- 7: Knochenmark
- 8: Taperung
- 9: Schneide der Kanüle
- 10: proximale Verbindungsfläche
- 19: Trokar
- 20: Griffstück
- 21: Griffstück
- 22: Griffstück Schaft 1
- B: Anschliffwinkel

## Patentansprüche

1. Biopsathaltevorrichtung für eine Biopsiekanüle (4) zur Ausführung transkutaner Biopsien von Gewebe, insbesondere hartem Gewebe und Knochenmarksgewebe, mit einer in das proximale Ende der Biopsiekanüle (4) einführbaren Biopsathaltevorrichtung, welche zwischen der Innenwand der Biopsiekanüle (4) und dem zu entnehmenden Gewebezylinder einschiebbar ist, **dadurch gekennzeichnet, dass** ein Draht (3) mit einem Anschliff (5) am distalen Ende des Drahtes (3) an der Biopsathaltevorrichtung angeordnet ist, wobei der Draht (3) einen Vorspannungswinkel (2) aufweist.

2. Biopsathaltevorrichtung für eine Biopsiekanüle (4) nach Anspruch 1 **dadurch gekennzeichnet, dass** sich die Biopsathaltevorrichtung daraus zusammensetzt, dass an einem Griffstück (22) der Draht (3) angeordnet ist.

3. Biopsathaltevorrichtung für eine Biopsiekanüle (4) nach Anspruch 1 **dadurch gekennzeichnet, dass** sich die Biopsathaltevorrichtung daraus zusammensetzt, dass an einem Griffstück (22) ein Schaft (1) zur Verlängerung angeordnet ist und an dem Schaft (1) der Draht (3) vorhanden ist.

4. Biopsathaltevorrichtung für eine Biopsiekanüle (4) nach Anspruch 1 **dadurch gekennzeichnet, dass** der Draht (3) eine Spitze mit einem Anschliff (5) aufweist, wobei die Spitze mit Anschliff (5) einen Anschliffwinkel B von 5 bis 85°, vorteilhafterweise 20°, aufweist und hin zum Biopsatzylinder (6) gerichtet ist.

5. Biopsathaltevorrichtung für eine Biopsiekanüle (4) nach den Ansprüchen 1 und 4 **dadurch gekennzeichnet, dass** der Anschliff (5) des Drahtes (3) einen Hohlschliff oder bauchig ausgestaltet ist.

6. Biopsathaltevorrichtung für eine Biopsiekanüle (4) nach Anspruch 1 **dadurch gekennzeichnet, dass** der Draht (3) mit einem Vorspannungswinkel (2) am Griffstück (22) mittig angeordnet ist, wobei der Vorspannungswinkel (2) zwischen 1 bis 90° vorhanden ist.

7. Biopsathaltevorrichtung für eine Biopsiekanüle (4) nach Anspruch 1 **dadurch gekennzeichnet, dass** der Draht (3) eine Länge aufweist, welche der Biopsiekanüle (4) entspricht und am unmittelbaren Ende endet.

8. Biopsathaltevorrichtung für eine Biopsiekanüle (4) nach Anspruch 1 **dadurch gekennzeichnet, dass** das Griffstück (22) arretierbar in das Griffstück (20) der Biopsiekanüle (4) vorgesehen ist.

9. Biopsathaltevorrichtung für eine Biopsiekanüle (4) nach Anspruch 3 **dadurch gekennzeichnet, dass** der Draht (3) an dem Schaft (1) am distalen Ende fest verbunden ist und ein Vorspannungswinkel (2) zwischen 1 und 90° je nach Anwendungsfall gegeben ist.

10. Biopsathaltevorrichtung für eine Biopsiekanüle (4) nach Anspruch 3 **dadurch gekennzeichnet, dass** die Länge des Drahtes (3) vorteilhafterweise 25 mm aufweist und am unmittelbaren Ende einer an sich bekannten Biopsiekanüle (4) nach Einführung des Schaftes (1) und Arretierung des Griffstückes (22) in den Griff (20) der Biopsiekanüle (4) endet.

11. Biopsathaltevorrichtung für eine Biopsiekanüle (4) nach Anspruch 1 **dadurch gekennzeichnet, dass** der Draht (3) an seiner Oberfläche aufgerauht ist und in einem beliebigen Profil ausgestaltet wird, wobei der Querschnitt des Drahtes (3) so dimensioniert ist, dass eine Fixierung des Drahtes (3) am distalen Ende der Biopsiekanüle (4) eine minimale Quetschartefaktur hervorruft, wobei der Durchmesser vorteilhafterweise ca. 0,35 mm ist.

12. Biopsathaltevorrichtung für eine Biopsiekanüle (4) nach Anspruch 3 **dadurch gekennzeichnet, dass** der Draht (3) seitlich ohne einen Vorspannungswinkel (2) an dem Schaft (1) angeordnet ist und somit an der Innenwand der Biopsiekanüle (4) anliegt.

## Claims

1. A biopsy material holding device for a biopsy cannula (4) to perform transcutaneous biopsies of tissue, in particular hard tissue and bone marrow tissue by means of a biopsy material holding device that can be inserted into the proximal end of a biopsy cannula (4) and is inserted between the inner wall of a biopsy cannula (4) and the tissue-removing cylinder, **characterised in that** a wire (3) with a bevelling (5) is arranged at the distal end of the wire (3) at a biopsy material holding device and the wire (3) having a Pre-stress angle (2).

2. A biopsy material holding device for a biopsy cannula (4) according to claim 1 **characterised in that** the biopsy material holding device is made of a wire (3) attached to a grip end (22).

3. A biopsy material holding device for a biopsy cannula (4) according to claim 1 **characterised in that that** the biopsy material holding device is composed of a grip end (22) with an attached extension shank (1) and that a wire (3) is fastened to the shank (1).

4. A biopsy material holding device for a biopsy cannula (4) according to claim 1 **characterised in that** the wire (3) has a tip with a bevelling (5) and the tip with bevelling (5) has a bevelling angle B of 5° to 85°, preferably 20°, and is facing the biopsy material cylinder (6).

5. A biopsy material holding device for a biopsy cannula (4) according to claims 1 and 4 **characterised in that** the bevelling (5) of the wire (3) is either hollow ground or bulged.

6. A biopsy material holding device for a biopsy cannula (4) according to claim 1 **characterised in that** the wire (3) with a pre-stress angle (2) is arranged at the centre of the grip end (22), with the pre-stress angle (2) being between 1° and 90°.

7. A biopsy material holding device for a biopsy cannula (4) according to claim 1 **characterised in that** the wire (3) has a length that corresponds to that of the biopsy cannula (4) at ends at the latter's ending.

8. A biopsy material holding device for a biopsy cannula (4) according to claim 1 **characterised in that** the grip end (22) can be locked into the grip end (20) of the biopsy cannula (4).

9. A biopsy material holding device for a biopsy cannula (4) according to claim 3 **characterised in that** the wire (3) is firmly connected to the distal end of the shank (1) and a pre-stress angle (2) between 1° and 90° is provided, according to the specific application.

10. A biopsy material holding device for a biopsy cannula (4) according to claim 3 **characterised in that** the length of the wire (3) is preferably 25 mm and ends at the direct end of a known biopsy cannula (4) after insertion of the shank (1) and locking of the grip end (22) into the grip (20) of the biopsy cannula (4).

11. A biopsy material holding device for a biopsy cannula (4) according to claim 1 **characterised in that** the wire (3) has a roughened surface and is shaped in an optional profile with the cross-section of the wire (3) being dimensioned in such a manner that fastening of the wire (3) at the distal end of the biopsy cannula (4) conditions a minimal pinch artefacture with a diameter of preferably ca. 0.35 mm.

12. A biopsy material holding device for a biopsy cannula (4) according to claim 3 **characterised in that** the wire (3) is arranged without a pre-stress angle (2) at the side of the shank (1) and thus fits closely to the interior wall of the biopsy cannula (4).

## Revendications

1. Dispositif de fixation de tissu prélevé dans le cadre d'une biopsie prévu à retenir une canule de biopsie (4) servant à effectuer des biopsies transcutanées, en particulier la biopsie de tissus rigides et de tissus myéloïdes, étant composé d'un dispositif de fixation de tissu prélevé dans le cadre d'une biopsie, celui-ci pouvant être introduit dans l'extrémité proximal de la canule de biopsie (4) appropriée, cependant, ledit dispositif de fixation de tissu prélevé dans le cadre d'une biopsie offrant la possibilité d'être introduit en glissant entre le paroi intérieur de la canule de biopsie (4) et le morceau cylindrique de tissu à prélever, étant **caractérisé par le fait qu'**un fil (3) celui-ci étant doté d'une coupe polie (5) se situant à l'extrémité distal du fil (3), est placé au niveau d'un dispositif de fixation de tissu prélevé dans le cadre d'une biopsie, cependant, ledit fil (3) présentant un certain angle de précontrainte (2).

2. Conformément à la revendication 1, le dispositif de fixation de tissu prélevé dans le cadre d'une biopsie prévu à retenir une canule de biopsie (4) étant **caractérisé par le fait que** le dispositif de fixation de tissu prélevé dans le cadre d'une biopsie se compose de manière qu'au niveau d'une manche (22) le fil (3) est placé.

3. Conformément à la revendication 1, le dispositif de fixation de tissu prélevé dans le cadre d'une biopsie prévu à retenir une canule de biopsie (4) étant **caractérisé par le fait que** le dispositif de fixation de tissu prélevé dans le cadre d'une biopsie se compose de manière qu'au niveau d'une manche (22) une tige (1) est placée servant de rallonge et qu'au niveau de ladite tige se trouve le fil (3).

4. Conformément à la revendication 1, le dispositif de fixation de tissu prélevé dans le cadre d'une biopsie prévu à retenir une canule de biopsie (4) **étant caractérisé par le fait que** le fil (3) présente une pointe, celle-ci étant dotée d'une coupe polie (5), cependant, la pointe dotée de ladite coupe polie (5) présentant un angle de la coupe polie B de 5° à 85°, cet angle de la coupe polie se situant à 20° d'avantage et étant orienté vers le morceau cylindrique de tissu à prélever dans le cadre d'une biopsie(6).

5. Conformément aux revendications 1 et 4, le dispositif de fixation de tissu prélevé dans le cadre d'une biopsie prévu à retenir une canule de biopsie (4) étant **caractérisé par le fait que** la coupe polie (5) se présentant au niveau du fil (3) a été réalisé sous forme d'un affûtage en coupe cuillère ou sous forme convexe après bombage.

6. Conformément à la revendication 1, le dispositif de fixation de tissu prélevé dans le cadre d'une biopsie prévu à retenir une canule de biopsie (4) étant **caractérisé par le fait que** le fil (3) présentant un certain angle de précontrainte (2) est placé de manière centrale au niveau de la manche (22), cependant, il y a présence d'un angle de précontrainte (2) se situant entre 1° et 90°.

7. Conformément à la revendication 1, le dispositif de fixation de tissu prélevé dans le cadre d'une biopsie prévu à retenir une canule de biopsie (4) étant **caractérisé par le fait que** le fil (3) présente une longueur qui correspond à la longueur de la canule de biopsie (4) et prend fin à son extrémité immédiat.

8. Conformément à la revendication 1, le dispositif de fixation de tissu prélevé dans le cadre d'une biopsie prévu à retenir une canule de biopsie (4) étant **caractérisé par le fait que** la manche (22) est prévu de sorte qu'elle présente la possibilité d'arrêtage au niveau de la manche (20) faisant partie de la canule de biopsie (4).

9. Conformément à la revendication 3, le dispositif de fixation de tissu prélevé dans le cadre d'une biopsie prévu à retenir une canule de biopsie (4) étant **caractérisé par le fait que** le fil (3) est relié à la tige (1) sous forme d'une liaison forte et cela au niveau de son extrémité distal et, qu'un angle de précontrainte (2) se situant entre 1° et 90° y présente en fonction du champ d'application en question.

10. Conformément à la revendication 3, le dispositif de fixation de tissu prélevé dans le cadre d'une biopsie prévu à retenir une canule de biopsie (4) étant **caractérisé par le fait que** la longueur du fil (3) est de 25 mm d'avantage et, que se termine à l'extrémité immédiat d'une canule de biopsie (4) connue après introduction de la tige (1) et arrêtage de la manche (22) au niveau de la manche (20) de la canule de biopsie (4).

11. Conformément à la revendication 1, le dispositif de fixation de tissu prélevé dans le cadre d'une biopsie prévu à retenir une canule de biopsie (4) étant **caractérisé par le fait que** le fil (3) dispose d'une surface qui a été rendue rugueuse et que le profil du fil est à choix libre, cependant, la coupe en travers du fil (3) étant dimensionné de sorte que la fixation du fil (3) au niveau de l'extrémité distal de la canule de biopsie (4) provoque un effort minimal d'écrasement, cependant, un diamètre de 0,35 mm environ étant préférable.

12. Conformément à la revendication 3, le dispositif de fixation de tissu prélevé dans le cadre d'une biopsie prévu à retenir une canule de biopsie (4) étant **caractérisé par le fait que** le fil (3) est placé de manière latérale au niveau de la tige (1) sans qu'un angle de précontrainte (2) y présente et le fil se localise très près du paroi intérieur de la canule de biopsie (4).
